# EUROPEAN PATENT SPECIFICATION

(11) **EP 2 777 644 B1**
(45) Date of publication and mention of the grant of the patent: **04.05.2016**
(21) Application number: 14159018.2
(22) Date of filing: 12.03.2014
(51) Int. Cl.: A61F 2/95

(54) **Striped stent introducer**
Gestreifter Stent-Einführer
Introducteur de stent avec une bande

(30) Priority: 13.03.2013 US 201313801283
(43) Date of publication of application: 17.09.2014
(73) Proprietor: DePuy Synthes Products, LLC, Raynham, MA 02767 (US)
(72) Inventor: Slazas, Robert, Pinecrest, FL Florida 33156 (US)
(74) Representative: Small, Gary James

(56) References cited:
- US-A- 6 149 680
- US-A1- 2009 082 840
- US-A1- 2009 259 287

## Description

### BACKGROUND OF THE INVENTION

This invention relates generally to devices for interventional therapeutic treatment or vascular surgery for treatment of defects in the vasculature, and more particularly concerns a system and method for delivering endovascular medical devices, such as for treatment of aneurysms.

When introducing an endovascular medical device into the proximal hub of a catheter (through which the device is intended to travel), there is an important step that can be difficult for the user to complete successfully. It is important that the user be able to visually inspect the device while it is enclosed in a tubular introducer sheath, to ensure that it is not deranged or damaged. It is also important that the user inserts the introducer sheath all the way to the bottom of the tapered opening of the proximal hub of the catheter, so that the device is pushed directly into the lumen of the catheter. However, when using introducer sheaths made of entirely clear material it is difficult to tell if they have been inserted all the way to the bottom of the tapered opening of the hub. Since endovascular procedures are typically done with "saline flush" (the catheter and hub are filled with water), introducers made of clear material tend to disappear when submerged and the edges are very hard to distinguish.

One attempt to solve this problem has involved adding a colorant to the entire material of the introducer sheath, making a sort of "tinted" appearance. This improves the ability to detect the end of the introducer sheath, but detracts from the user's ability to inspect the device through the introducer sheath. In fact, the two requirements are in direct competition. The easier to inspect, the harder to detect the introducer sheath, and vice versa.

Another attempt to solve this problem involved adding a colored tip to the introducer sheath. This leaves a large portion of the introducer sheath clear for inspection, while coloring a distal section for detection. This approach takes the two requirements out of competition with each other, but adds a new risk of tip separation and additional cost of manufacturing the discrete tip onto the introducer sheath body.

It would be desirable to provide a tubular introducer sheath that allows a user to determine if the distal end of the introducer sheath has been inserted all the way to the bottom of a tapered hub opening, particularly in wet environments, and allows a user to visually inspect an endovascular device disposed within the introducer sheath prior to use of the endovascular device. It would also be desirable to provide a tubular introducer sheath that allows a user to visually inspect any features of interest on such an endovascular device within the introducer sheath by axially sliding the introducer sheath so that the features of interest can be seen, without having to rotate the introducer sheath to inspect the endovascular device. The present invention meets these and other needs.
US 2009/0082840 discloses a tubular introducer sheath for introducing a stent into a catheter. The sheath includes a hollow, elongated tubular member having a proximal end, a distal end, a length extending between the proximal and distal ends, an outer side wall, a longitudinal axis, and a longitudinal interior channel configured to removably contain a stent therewithin. The outer wall may be made from a transparent extrusion material. The outer wall may have a marker therein, in the form of a circumferential ring, to indicate the distal position of a stent as it is inserted into the introducer.

### SUMMARY OF THE INVENTION

Briefly and in general terms, the present invention provides for a tubular introducer sheath made of predominantly clear material, with a colored stripe that extends along the length of the tubular introducer sheath and that in a presently preferred aspect, spirals along the length of the tubular introducer sheath around the longitudinal axis of the tubular introducer sheath.

The present invention accordingly provides for a tubular introducer sheath for introducing an endovascular medical device into a catheter. In a presently preferred embodiment, the tubular introducer sheath includes a hollow, elongated tubular member having an outer side wall with a predominantly substantially clear portion, and a colored stripe embedded in the substantially clear portion of the outer side wall of the hollow, elongated tubular member along the length of the hollow, elongated tubular member. In a presently preferred aspect, the colored stripe spirals around the hollow, elongated tubular member and along the longitudinal axis of the hollow, elongated tubular member. In another presently preferred aspect, the colored stripe has a width that is sufficiently narrow that the clear area allows visual inspection through the tubular introducer sheath. In another presently preferred aspect, the distal end portion of the hollow, elongated tubular member is tapered.

The present invention also provides for a system for introducing an endovascular medical device into a catheter, including a hollow, elongated tubular introducer sheath having an outer side wall with a predominantly substantially clear portion, and a colored stripe embedded in the substantially clear portion of the outer side wall of the hollow, elongated tubular introducer sheath along the length of the hollow, elongated tubular introducer sheath, and an endovascular medical device removably disposed within the hollow, elongated tubular introducer sheath. In a presently preferred aspect, the colored stripe spirals around the hollow, elongated tubular member and along the longitudinal axis of the hollow, elongated tubular introducer sheath. In another presently preferred aspect, the colored stripe has a width that is sufficiently narrow that the clear area allows visual inspection through the hollow, elongated tubular introducer sheath. Inspection of a device within the hollow, elongated tubular introducer sheath through the clear portions of the introducer sheath is easily done by axially sliding the introducer sheath so that features of interest are not blocked by the colored stripe, without the need to rotate the hollow, elongated tubular introducer sheath to inspect the device. In another presently preferred aspect, the distal end portion of the hollow, elongated tubular introducer sheath is tapered.

Other features and advantages of the present invention will become more apparent from the following detailed description of the preferred embodiments in conjunction with the accompanying drawings, which illustrate, by way of example, the operation of the invention.

### BRIEF DESCRIPTION OF THE DRAWINGS

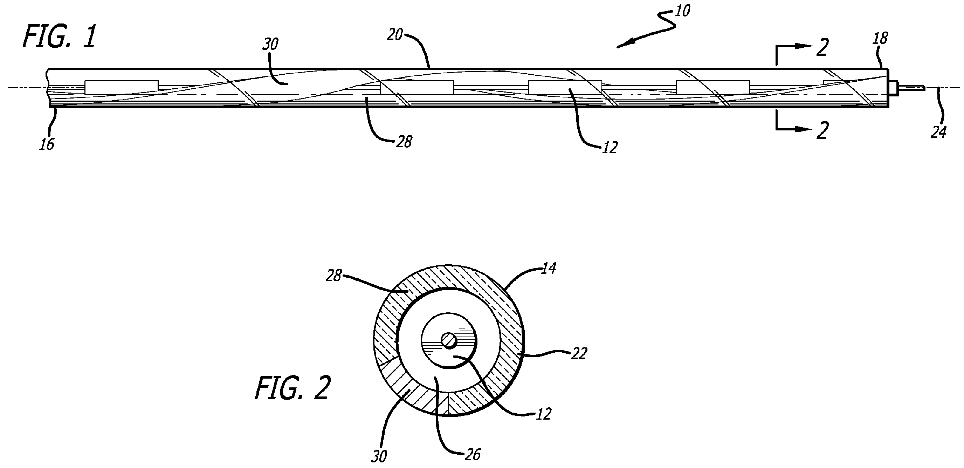

Figure 1 is a side elevational view of a striped tubular stent introducer sheath, according to the invention.
Fig. 2 is a cross-sectional view taken along line 2-2 of Fig. 1.

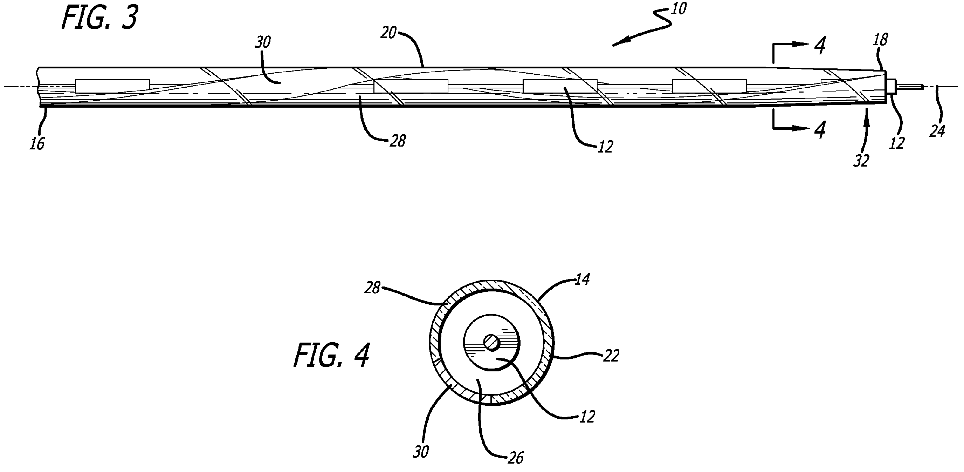

Fig. 3 is a side elevational view of a variation of the striped tubular stent introducer sheath of Fig. 1.
Fig. 4 is a cross-sectional view taken along line 4-4 of Fig. 3.

### DETAILED DESCRIPTION OF THE PREFERRED EMBODIMENTS

Referring to the drawings, which are provided by way of example, and not by way of limitation, the present invention provides for a tubular introducer sheath 10 for introducing an endovascular medical device 12 into a catheter (not shown). The tubular introducer sheath is preferably formed as a hollow, elongated tubular member 14 having a proximal end 16, a distal end 18, and a length 20 extending between the proximal and distal ends. The tubular introducer sheath includes an outer side wall 22, a longitudinal axis 24, and a longitudinal interior channel 26 configured to removably contain the endovascular medical device therewithin, for introducing the endovascular medical device into a catheter.

In a presently preferred aspect, the tubular introducer sheath is made of predominantly clear material, such as a clear plastic or polymeric material, such as polytetrafluoroethylene (PTFE) or polycarbonate, for example, forming a clear portion 28 with a colored stripe 30 embedded in the outer side wall of the hollow, elongated tubular member along the length of the hollow, elongated tubular member.

The colored stripe is preferably formed to spiral around along the longitudinal axis of the tubular introducer sheath. During extrusion of the tubular introducer sheath, a discrete sector of the circumference is displaced by a stream of colored material. As the extrusion proceeds, the material is rotated so that the stripe of colored material travels around the tube. The width of the stripe preferably is formed to be sufficiently wide to be visible externally, and sufficiently narrow that the clear portion of the tubular introducer sheath allows visual inspection of an endovascular medical device disposed in the tubular introducer sheath through the clear portion of the tubular introducer sheath. The presence of the colored stripe helps the user determine if the end of the tubular introducer sheath has been inserted all the way to the bottom of the tapered hub opening.

In another presently preferred aspect, in one variation, a distal end portion 32 of the tubular introducer sheath can be tapered, such as to match an internal taper of a catheter hub through which the endovascular medical device may be introduced into a catheter.

As is illustrated in the drawings, when a sample device is within the spiral-striped introducer sheath, the ends of the introducer sheath are clearly marked by the termination of the colored stripe. Inspecting the device through the clear portions of the introducer sheath is easily done by axially sliding the introducer sheath so that the features of interest are not blocked by the stripe, and it is not necessary to rotate the introducer sheath to inspect the device.

It will be apparent from the foregoing that while particular forms of the invention have been illustrated and described, various modifications can be made without departing from the scope of the invention. Accordingly, it is not intended that the invention be limited, except as by the appended claims.

## Claims

1. A tubular introducer sheath (10) for introducing an endovascular medical device (12) into a catheter, the tubular introducer sheath comprising:
a hollow, elongated tubular member (14) having a proximal end (16), a distal end (18), a length (20) extending between the proximal and distal ends (16,18), an outer side wall (22), a longitudinal axis (24), and a longitudinal interior channel (26) configured to removably contain an endovascular medical device (12) therewithin, said outer side wall (22) having a predominantly substantially clear portion (28); and
a longitudinal colored stripe (30) embedded in the substantially clear portion (28) of said outer side wall (22) of the hollow, elongated tubular member (14) along the length of the hollow, elongated tubular member (14).

2. The tubular introducer sheath (10) of Claim 1, wherein said colored stripe (30) spirals around the hollow, elongated tubular member (14) and along the longitudinal axis of the hollow, elongated tubular member (14).

3. The tubular introducer sheath (10) of Claim 1, wherein said colored stripe (30) has a width that is sufficiently narrow that the clear area (28) allows visual inspection through the tubular introducer sheath (10).

4. The tubular introducer sheath (10) of Claim 1, wherein a distal end (18) portion of the hollow, elongated tubular member (14) is tapered.

5. A system for introducing an endovascular medical device into a catheter, comprising:
a hollow, elongated tubular introducer sheath (10) having a proximal end (16), a distal end (18), a length (20) extending between the proximal and distal ends (16, 18), an outer side wall (22), a longitudinal axis (24), and a longitudinal interior channel (26), said outer side wall (22) having a predominantly substantially clear portion (28);
a longitudinal colored stripe (30) embedded in the substantially clear portion (28) of said outer side wall (22) of the hollow, elongated tubular introducer sheath (10) along the length of the hollow, elongated tubular introducer sheath (10); and
an endovascular medical device (12) removably disposed within said hollow, elongated tubular introducer sheath (10).

6. The system of Claim 5, wherein said colored stripe (30) spirals around the hollow, elongated tubular introducer sheath (10) and along the longitudinal axis of the hollow, elongated tubular introducer sheath (10).

7. The system of Claim 5, wherein said colored stripe (30) has a width that is sufficiently narrow that the clear area (28) allows visual inspection through the hollow, elongated tubular introducer sheath (10).

8. The system of Claim 5, wherein a distal end (18) portion of the hollow, elongated tubular introducer sheath (10) is tapered.

## Patentansprüche

1. Rohrförmige Einführerhülse (10) zum Einführen einer endovaskulären medizinischen Vorrichtung (12) in einen Katheter, wobei die rohrförmige Einführerhülse Folgendes umfasst:
ein hohles längliches rohrförmiges Element (14) mit einem proximalen Ende (16), einem distalen Ende (18), einer sich zwischen dem proximalen und dem distalen Ende (16, 18) erstreckenden Länge (20), einer äußeren Seitenwand (22), einer Längsachse (24) und einem länglichen inneren Kanal (26), der dazu konfiguriert ist, darin eine endovaskuläre medizinische Vorrichtung (12) entnehmbar zu enthalten, wobei die äußere Seitenwand (22) einen vorwiegend im Wesentlichen klaren Abschnitt (28) hat, und
einen länglichen farbigen Streifen (30), der in dem im Wesentlichen klaren Abschnitt (28) der äußeren Seitenwand (22) des hohlen länglichen rohrförmigen Elements (14) entlang der Länge des hohlen länglichen rohrförmigen Elements (14) eingebettet ist.

2. Rohrförmige Einführerhülse (10) nach Anspruch 1, wobei der farbige Streifen (30) spiralförmig um das hohle längliche rohrförmige Element (14) und entlang der Längsachse des hohlen länglichen rohrförmigen Elements (14) läuft.

3. Rohrförmige Einführerhülse (10) nach Anspruch 1, wobei der farbige Streifen (30) eine Breite hat, die ausreichend schmal ist, dass der klare Bereich (28) die visuelle Einsichtnahme durch die rohrförmige Einführerhülse (10) gestattet.

4. Rohrförmige Einführerhülse (10) nach Anspruch 1, wobei sich ein Abschnitt des distalen Endes (18) des hohlen länglichen rohrförmigen Elements (14) verjüngt.

5. System zum Einführen einer endovaskulären medizinischen Vorrichtung in einen Katheter, umfassend:
eine hohle längliche rohrförmige Einführerhülse (10) mit einem proximalen Ende (16), einem distalen Ende (18), einer sich zwischen dem proximalen und dem distalen Ende (16, 18) erstreckenden Länge (20), einer äußeren Seitenwand (22), einer Längsachse (24) und einem länglichen inneren Kanal (26), wobei die äußere Seitenwand (22) einen vorwiegend im Wesentlichen klaren Abschnitt (28) hat,
einen länglichen farbigen Streifen (30), der in dem im Wesentlichen klaren Abschnitt (28) der äußeren Seitenwand (22) der hohlen länglichen rohrförmigen Einführerhülse (10) entlang der Länge der hohlen länglichen rohrförmigen Einführerhülse (10) eingebettet ist, und
eine endovaskuläre medizinische Vorrichtung (12), die entnehmbar in der hohlen länglichen rohrförmigen Einführerhülse (10) angeordnet ist.

6. System nach Anspruch 5, wobei der farbige Streifen (30) spiralförmig um die hohle längliche rohrförmige Einführerhülse (10) und entlang der Längsachse der hohlen länglichen rohrförmigen Einführerhülse (10) läuft.

7. System nach Anspruch 5, wobei der farbige Streifen (30) eine Breite hat, die ausreichend schmal ist, dass der klare Bereich (28) die visuelle Einsichtnahme durch die hohle längliche rohrförmige Einführerhülse (10) gestattet.

8. System nach Anspruch 5, wobei sich ein Abschnitt des distalen Endes (18) der hohlen länglichen rohrförmigen Einführerhülse (10) verjüngt.

## Revendications

1. Gaine d'introduction tubulaire (10) permettant l'introduction d'un dispositif médical endovasculaire (12) dans un cathéter, la gaine d'introduction tubulaire comprenant :
un élément creux, allongé et tubulaire (14) comportant une extrémité proximale (16), une extrémité distale (18), une longueur (20) s'étendant entre les extrémités proximale et distale (16, 18),
une paroi latérale extérieure (22), un axe longitudinal (24) et un canal longitudinal intérieur (26) configuré pour contenir à l'intérieur de lui, de manière amovible, un dispositif médical endovasculaire (12), ladite paroi latérale extérieure (22) comportant une partie en majorité essentiellement transparente (28) ; et
une rayure longitudinale colorée (30) intégrée dans la partie essentiellement transparente (28) de ladite paroi latérale extérieure (22) de l'élément creux, allongé et tubulaire (14) le long de la longueur de l'élément creux, allongé et tubulaire (14).

2. Gaine d'introduction tubulaire (10) selon la revendication 1, dans laquelle ladite rayure colorée (30) s'étend en spirale autour de l'élément creux, allongé et tubulaire (14) et le long de l'axe longitudinal de l'élément creux, allongé et tubulaire (14).

3. Gaine d'introduction tubulaire (10) selon la revendication 1, dans laquelle ladite rayure colorée (30) présente une largeur qui est suffisamment faible pour que la région transparente (28) permette une inspection visuelle à travers la gaine d'introduction tubulaire (10).

4. Gaine d'introduction tubulaire (10) selon la revendication 1, dans laquelle une partie d'extrémité distale (18) de l'élément creux, allongé et tubulaire (14) est effilée.

5. Système d'introduction d'un dispositif médical endovasculaire dans un cathéter, comprenant :
une gaine d'introduction creuse, allongée et tubulaire (10) comportant une extrémité proximale (16), une extrémité distale (18), une longueur (20) s'étendant entre les extrémités proximale et distale (16, 18), une paroi latérale extérieure (22), un axe longitudinal (24) et un canal longitudinal intérieur (26), ladite paroi latérale extérieure (22) comportant une partie en majorité essentiellement transparente (28) ;
une rayure longitudinale colorée (30) intégrée dans la partie essentiellement transparente (28) de ladite paroi latérale extérieure (22) de la gaine d'introduction creuse, allongée et tubulaire (10) le long de la longueur de la gaine d'introduction creuse, allongée et tubulaire (10) ; et
un dispositif médical endovasculaire (12) disposé, de manière amovible, à l'intérieur de ladite gaine d'introduction creuse, allongée et tubulaire (10).

6. Système selon la revendication 5, dans lequel ladite rayure colorée (30) s'étend en spirale autour de la gaine d'introduction creuse, allongée et tubulaire (10) et le long de l'axe longitudinal de la gaine d'introduction creuse, allongée et tubulaire (10).

7. Système selon la revendication 5, dans lequel ladite rayure colorée (30) présente une largeur qui est suffisamment faible pour que la région transparente (28) permette une inspection visuelle à travers la gaine d'introduction creuse, allongée et tubulaire (10).

8. Système selon la revendication 5, dans lequel une partie d'extrémité distale (18) de la gaine d'introduction creuse, allongée et tubulaire (10) est effilée.
